# EUROPÄISCHE PATENTSCHRIFT

(11) **EP 3 696 149 B1**
(45) Veröffentlichungstag und Bekanntmachung des Hinweises auf die Patenterteilung: **11.02.2026**
(21) Anmeldenummer: 19157303.9
(22) Anmeldetag: 14.02.2019
(51) Int. Cl.: A61K 6/833, A61K 6/836, C03C 4/12, C03C 4/00, C03C 3/095, C03C 3/097, C03C 8/04, C03C 8/06, C03C 8/08, A61C 13/08, A61K 6/816, C03C 3/112, C03C 10/00, C03C 8/14, A61K 6/804, A61K 6/822

(54) **FLUORESZIERENDE GLASKERAMIKEN UND GLÄSER MIT GEHALT AN CER UND ZINN**
FLUORESCENT GLASS CERAMICS AND GLASSES CONTAINING CERIUM AND TIN
VITROCÉRAMIQUE FLUORESCENTE ET VERRES CONTENANT DU CÉRIUM ET DE L'ÉTAIN

(43) Veröffentlichungstag der Anmeldung: 19.08.2020
(73) Patentinhaber: Ivoclar Vivadent AG, 9494 Schaan (LI)
(72) Erfinder: Hengst, Ronny, 9470 Buchs (CH); Dittmer, Marc, 6800 Feldkirch (AT)
(74) Vertreter: Uexküll & Stolberg

(56) Entgegenhaltungen:
- EP-A1- 0 916 625
- EP-A1- 1 870 384
- EP-A1- 1 905 746
- WO-A1-2014/081454
- US-A1- 2010 083 706

## Beschreibung

Die vorliegende Erfindung betrifft Glaskeramiken und Gläser, die Cer und Zinn enthalten und sich insbesondere zur Herstellung von Dentalrestaurationen eignen, deren Fluoreszenzeigenschaften weitgehend denen der natürlichen Zähne entsprechen. Die Erfindung betrifft auch ein Verfahren zur Herstellung der erfindungsgemäßen Glaskeramiken und Gläser sowie deren Verwendung als Dentalmaterial und insbesondere zur Herstellung dentaler Restaurationen.

Glaskeramiken werden in der Zahnheilkunde aufgrund ihrer guten mechanischen und optischen Eigenschaften insbesondere zur Herstellung von Dentalkronen und kleinen Brücken eingesetzt.

EP 0 916 625 A1 beschreibt Lithiumdisilikat-Glaskeramiken, die Lithiumdisilikat als Hauptkristallphase enthalten und aufgrund ihrer hohen Transluzenz und sehr guten mechanischen Eigenschaften besonders im Dentalbereich und dabei vornehmlich zur Herstellung von Kronen und Brücken Anwendung finden. Um die Farbe der Glaskeramik-Produkte an die Farbe des natürlichen Zahnmaterials anzupassen, können die Glaskeramiken Farb- und Fluoreszenzkomponenten aufweisen, welche vorzugsweise aus der Gruppe bestehend aus CeO₂, V₂O₅, Fe₂O₃, MnO₂, TiO₂, Y₂O₃, Er₂O₃, Tb₄O₇, Eu₂O₃, Yb₂O₃, Gd₂O₃, Nd₂O₃, Pr₂O₃, Dy₂O₃, Ag₂O, SnO₂ und Ta₂O₅ ausgewählt sind.

WO 2015/173394 A1 beschreibt Glaskeramiken, die SiO₂ als Hauptkristallphase aufweisen und sich ebenfalls zur Herstellung von dentalen Restaurationen eignen. Als Färbemittel oder Fluoreszenzmittel können diese Glaskeramiken insbesondere Oxide von Sc, Mn, Fe, Co, Pr, Nd, Tb, Er, Dy, Gd, Eu und Yb enthalten.

Aus W. Buchalla, "Comparative Fluorescence Spectroscopy Shows Differences in Non-Cavitated Enamel Lesions", Caries Res. 2005, 39, 150-156 ist bekannt, dass natürliche Zähne unter ultraviolettem Licht eine bläulichweisse Fluoreszenz mit Wellenlängen im Bereich von 400 bis 650 nm zeigen.

Rukmani et al., J. Am. Ceram. Soc. 2007, 90, 706-711, beschreiben den Einfluss von V- und Mn-Färbemitteln auf das Kristallisationsverhalten und die optischen Eigenschaften von Ce-dotierten Lithiumdisilikat-Glaskeramiken. Zur Herstellung der Glaskeramiken wird eine Mischung aus den Ausgangsmaterialien SiO₂, ZrO₂, Li₂CO₃, K₂CO₃, MgCO₃ und Al(PO₃)₃ mit CeO₂, V₂O₅ und MnO₂ hergestellt, die Mischung bei 1500°C in Platintiegeln geschmolzen, abgekühlt und anschließend in einem Röhrenofen unter Luftzufuhr mehreren Wärmebehandlungen unterzogen.

WO 2014/081454 A1 und US 2010/083706 A1 beschreiben Glaskeramiken zur Herstellung von dentalen Restaurationen, die unter anderem Cer und Zinn enthalten können.

Es hat sich jedoch gezeigt, dass die aus dem Stand der Technik bekannten Gläser und Glaskeramiken unzureichende Fluoreszenzeigenschaften aufweisen und insbesondere unter UV-Licht die Fluoreszenzeigenschaften des natürlichen Zahnmaterials nicht in ausreichendem Maße imitieren können. Dadurch werden aus solchen Glaskeramiken hergestellte Dentalrestaurationen insbesondere unter dem Einfluss von UV-Licht als Restaurationen erkennbar oder werden als Zahnlücken oder Defekte wahrgenommen.

Verfahren zur Herstellung von Gläsern und Glaskeramiken mit verbesserten Fluoreszenzeigenschaften sind ebenfalls beschrieben worden.

So beschreibt WO 2015/173230 A1 ein Verfahren zur Herstellung eines Lithiumsilikatglases oder einer Lithiumsilikat-Glaskeramik, bei dem eine Schmelze eines Ausgangsglases, das Cer-Ionen enthält, reduzierenden Bedingungen ausgesetzt wird. Dadurch sollen in dem Ausgangsglas enthaltene Ce⁴⁺-Ionen ganz oder teilweise zu Ce³⁺-Ionen reduziert werden, die aufgrund von 5d→4f-Übergängen eine Fluoreszenz im Wellenlängenbereich von 320 bis 500 nm zeigen. Ein entsprechendes Verfahren zur Herstellung einer Glaskeramik mit SiO₂ als Hauptkristallphase oder eines Glases, das Keime für die Kristallisation von SiO₂ enthält, ist aus WO 2017/080853 A1 bekannt.

Nachteilig an den bekannten Verfahren ist jedoch, dass sich das Verhältnis von Ce³⁺-Ionen und Ce⁴⁺-Ionen nur bedingt steuern lässt. Außerdem kann es bei den so hergestellten Gläsern und Glaskeramiken durch Wärmebehandlungen unter oxidierenden Bedingungen, beispielsweise beim Sintern, zu einer Verschiebung dieses Verhältnisses zugunsten von Ce⁴⁺-Ionen kommen, wodurch die Fluoreszenzeigenschaften signifikant beeinträchtigt werden können.

Der Erfindung liegt die Aufgabe zugrunde, Glaskeramiken und Gläser bereitzustellen, die eine mit dem natürlichen Zahnmaterial vergleichbare und gegenüber Wärmebehandlungen und oxidierenden Bedingungen weitgehend unempfindliche Fluoreszenz zeigen und sich somit insbesondere zur Herstellung von dentalen Restaurationen eignen, die nicht nur gute mechanische Eigenschaften aufweisen, sondern auch die Fluoreszenzeigenschaften des natürlichen Zahnmaterials bei Anregungswellenlängen im gesamten relevanten UV-Bereich weitgehend imitieren können. Insbesondere sollen sich die Glaskeramiken und Gläser auch als Mischungskomponenten zur Einstellung der Fluoreszenzeigenschaften anderer Gläser und Glaskeramiken eignen.

Diese Aufgabe wird erfindungsgemäß gelöst durch ein Glas oder eine Glaskeramik mit Gehalt an Cer und Zinn, die die folgenden Komponenten enthalten:

| Komponente | Gew.-% |
|---|---|
| SiO₂ | 59,0 bis 80,0 |
| Al₂O₃ | 0,1 bis 42,0 |
| Cer, berechnet als CeO₂ | 0,5 bis 10,0 |
| Zinn, berechnet als SnO | 0,1 bis 4,0 |
| B₂O₃ weiteres Oxid vierwertiger | 0 bis 5,0 |
| Elemente Me^{IV}O₂ | 0 bis 4, 0, |

wobei der Begriff "weiteres Oxid vierwertiger Elemente Me^{IV}O₂" vierwertige Oxide mit Ausnahme von SiO₂, CeO₂, SnO₂ und TiO₂ bezeichnet und dieses Me^{IV}O₂ insbesondere ausgewählt ist aus ZrO₂ und/oder GeO₂ und
wobei das molare Verhältnis von Cer, berechnet als CeO₂, zu Zinn, berechnet als SnO, im Bereich von 10:1 bis 1:1 liegt.

Es hat sich überraschend gezeigt, dass das erfindungsgemäße Glas und die erfindungsgemäße Glaskeramik gegenüber dem Stand der Technik verbesserte Fluoreszenzeigenschaften insbesondere unter Einwirkung von UV-Licht zeigen, die genau und reproduzierbar einstellbar und weitgehend stabil gegenüber Wärmebehandlungen und oxidierenden Bedingungen sind.

Ohne Beschränkung auf eine bestimmte Theorie wird angenommen, dass es in den erfindungsgemäßen Gläsern und Glaskeramiken zur Ausbildung eines Gleichgewichts zwischen Ce³⁺/Ce⁴⁺ und Sn²⁺/Sn⁴⁺ kommt. Dadurch wird das Verhältnis von Ce³⁺-Ionen und Ce⁴⁺-Ionen stabilisiert und eine unerwünschte Verschiebung dieses Verhältnisses hin zu Ce⁴⁺-Ionen etwa bei Wärmebehandlungen insbesondere unter oxidierenden Bedingungen weitgehend verhindert. Die Ce³⁺-Ionen zeigen aufgrund von 5d→4f-Übergängen eine Fluoreszenz im Wellenlängenbereich von 320 bis 500 nm, die in besonderer Weise geeignet ist, die Fluoreszenzeigenschaften des natürlichen Zahnmaterials zu imitieren. Zudem bewirken Ce⁴⁺-Ionen eine Gelbfärbung der Gläser und Glaskeramiken. Somit wird eine besonders gute Imitation der Fluoreszenz- und Farbeigenschaften des natürlichen Zahnmaterials ermöglicht.

Erfindungsgemäß ist es bevorzugt, dass das Glas und die Glaskeramik 59,0 bis 77,0, insbesondere 59,0 bis 76,0, bevorzugt 64,0 bis 75,0 und besonders bevorzugt 70,0 bis 74,0 Gew.-% SiO₂ enthalten.

Weiter ist es bevorzugt, dass das Glas und die Glaskeramik 0,3 bis 39,0, insbesondere 0,5 bis 30,0, bevorzugt 1,0 bis 20,0, besonders bevorzugt 1,5 bis 10,0 und am meisten bevorzugt 2,0 bis 6,0 Gew.-% Al₂O₃ enthalten.

Vorzugsweise enthalten das Glas und die Glaskeramik 0,7 bis 7,5, insbesondere 1,0 bis 7,0, bevorzugt 1,5 bis 5,0 und besonders bevorzugt 2,0 bis 4,0 Gew.-% Cer, berechnet als CeO₂.

Es ist weiter bevorzugt, dass das Glas und die Glaskeramik 0,2 bis 3,0, insbesondere 0,3 bis 2,0 und bevorzugt 0,4 bis 1,0 Gew.-% Zinn, berechnet als SnO, enthalten.

Es ist außerdem bevorzugt, dass das molare Verhältnis von Cer, berechnet als CeO₂, zu Zinn, berechnet als SnO, im Bereich von 5:1 bis 1:1, bevorzugt 3:1 bis 1:1 und besonders bevorzugt 2:1 bis 1:1 liegt.

In einer besonderen Ausführungsform enthalten das Glas und die Glaskeramik ferner Terbium. Bevorzugt enthalten das Glas und die Glaskeramik 0 bis 2,0, insbesondere 0,05 bis 1,5, bevorzugt 0,1 bis 1,0 und besonders bevorzugt 0,3 bis 0,7 Gew.-% Terbium, berechnet als Tb₄O₇. Es hat sich überraschend gezeigt, dass erfindungsgemäß durch Kombination von Cer-Ionen und Terbium-Ionen Gläser und Glaskeramiken erhalten werden können, deren Fluoreszenz-und Farbeigenschaften die des natürlichen Zahnmaterials besonders gut imitieren können. Besonders überraschend ist dabei, dass bei den erfindungsgemäßen Gläsern und Glaskeramiken die durch die Cer-Ionen hervorgerufene Fluoreszenz auch in Anwesenheit von Terbium-Ionen weitgehend bestehen bleibt, obwohl im Stand der Technik eine Verringerung oder gar Auslöschung der durch Cer-Ionen hervorgerufenen Fluoreszenz bei Anwesenheit von d-Elementen beobachtet wurde.

Es ist weiter bevorzugt, dass das Glas und die Glaskeramik 0 bis 18,0, insbesondere 1,0 bis 17,0, bevorzugt 3,0 bis 16,0 und besonders bevorzugt 7,5 bis 10,0 Gew.-% Li₂O enthalten. Li₂O dient insbesondere der Verbesserung der Erschmelzbarkeit der Ausgangsgläser.

Auch ist es bevorzugt, dass das Glas und die Glaskeramik weiteres Alkalimetalloxid Me^{I}₂O in einer Menge 0 bis 13,0, insbesondere 1,0 bis 7,0 und bevorzugt 3,0 bis 5,0 Gew.-% enthalten. Der Begriff "weiteres Alkalimetalloxid Me^{I}₂O" bezeichnet Alkalimetalloxid mit Ausnahme von Li₂O, wobei dieses Me^{I}₂O insbesondere ausgewählt ist aus Na₂O, K₂O, Rb₂O und/oder Cs₂O, bevorzugt ausgewählt ist aus Na₂O und/oder K₂O und besonders bevorzugt K₂O ist. Besonders bevorzugt enthalten das Glas und die Glaskeramik mindestens eines und insbesondere alle der folgenden weiteren Alkalimetalloxide Me^{I}₂O in den angegebenen Mengen:

| | |
|---|---|
| Komponente | Gew.-% |
| Na₂O | 0 bis 8,0 |
| K₂O | 0 bis 5,0 |
| Rb₂O | 0 bis 7,0 |
| Cs₂O | 0 bis 13,0. |

Des Weiteren ist es bevorzugt, dass das Glas und die Glaskeramik 0 bis 22,0, insbesondere 1,0 bis 16,0, bevorzugt 2,0 bis 10,0 und besonders bevorzugt 3,0 bis 6,0 Gew.-% weiteres Oxid zweiwertiger Elemente Me^{II}O enthalten. Der Begriff "weiteres Oxid zweiwertiger Elemente Me^{II}O" bezeichnet zweiwertige Oxide mit Ausnahme von BaO und SnO, wobei dieses Me^{II}O insbesondere ausgewählt ist aus MgO, CaO, SrO und/oder ZnO. Besonders bevorzugt enthalten das Glas und die Glaskeramik mindestens eines und insbesondere alle der folgenden Oxide zweiwertiger Elemente Me^{II}O in den angegebenen Mengen:

| Komponente | Gew.-% |
|---|---|
| MgO | 0 bis 13, 0 |
| CaO | 0 bis 4,0 |
| SrO | 0 bis 3,0 |
| ZnO | 0 bis 4,0. |

Weiterhin ist es bevorzugt, dass das Glas und die Glaskeramik 0 bis 10,0, insbesondere 0 bis 5,0 und bevorzugt 0 bis 1,0 Gew.-% BaO enthalten und am meisten bevorzugt im Wesentlichen frei von BaO sind.

Es sind weiter ein Glas und eine Glaskeramik bevorzugt, die 0 bis 10,0, insbesondere 0,5 bis 4,0 und bevorzugt 1,0 bis 2,5 Gew.-% weiteres Oxid dreiwertiger Elemente Me^{III}₂O₃ enthalten. Der Begriff "weiteres Oxid dreiwertiger Elemente Me^{III}₂O₃" bezeichnet dreiwertige Oxide mit Ausnahme von Al₂O₃ und Ce₂O₃, wobei dieses Me^{III}₂O₃ insbesondere ausgewählt ist aus B₂O₃, Y₂O₃, La₂O₃, Ga₂O₃ und/oder In₂O₃ und bevorzugt ausgewählt ist aus B₂O₃, Y₂O₃ und/oder La₂O₃. Besonders bevorzugt enthalten das Glas und die Glaskeramik mindestens eines und insbesondere alle der folgenden weiteren Oxide dreiwertiger Elemente Me^{III}₂O₃ in den angegebenen Mengen:

| Komponente | Gew.-% |
|---|---|
| B₂O₃ | 0 bis 5, 0 |
| Y₂O₃ | 0 bis 3, 0 |
| La₂O₃ | 0 bis 2, 0 |
| Ga₂O₃ | 0 bis 2, 0 |
| In₂O₃ | 0 bis 1,0. |

Ferner können das Glas und die Glaskeramik weiteres Oxid vierwertiger Elemente Me^{IV}O₂ in einer Menge von 0 bis 4,0, insbesondere 0,5 bis 4,0 und bevorzugt 1,0 bis 2,5 Gew.-% enthalten. Der Begriff "weiteres Oxid vierwertiger Elemente Me^{IV}O₂" bezeichnet vierwertige Oxide mit Ausnahme von SiO₂, CeO₂, SnO₂ und TiO₂, wobei dieses Me^{IV}O₂ insbesondere ausgewählt ist aus ZrO₂ und/oder GeO₂. Besonders bevorzugt enthalten das Glas und die Glaskeramik mindestens eines und insbesondere alle der folgenden weiteren Oxide vierwertiger Elemente Me^{IV}O₂ in den angegebenen Mengen:

| Komponente | Gew.-% |
|---|---|
| ZrO₂ | 0 bis 4, 0 |
| GeO₂ | 0 bis 4, 0. |

Weiterhin ist es bevorzugt, dass das Glas und die Glaskeramik 0 bis 5,0, insbesondere 0 bis 2,5 und bevorzugt 0 bis 1,0 Gew.-% TiO₂ enthalten und am meisten bevorzugt im Wesentlichen frei von TiO₂ sind.

In einer bevorzugten Ausführungsform enthalten das Glas und die Glaskeramik ferner Oxid fünfwertiger Elemente Me^{V}₂O₅ in einer Menge von 0 bis 8,0, insbesondere 1,0 bis 6,0, bevorzugt 2,0 bis 5,0 und besonders bevorzugt 3,0 bis 4,0 Gew.-% Gew.-%, wobei dieses Me^{V}₂O₅ insbesondere ausgewählt ist aus P₂O₅, V₂O₅, Ta₂O₅ und/oder Nb₂O₅, bevorzugt ausgewählt ist aus P₂O₅ und/oder Ta₂O₅ und besonders bevorzugt P₂O₅ ist. Dabei kann P₂O₅ insbesondere als Keimbildner fungieren. Das Vorhandensein eines Keimbildners ist jedoch erfindungsgemäß nicht zwingend erforderlich. Besonders bevorzugt enthalten das Glas und die Glaskeramik mindestens eines und insbesondere alle der folgenden weiteren Oxide fünfwertiger Elemente Me^{V}₂O₅ in den angegebenen Mengen:

| Komponente | Gew.-% |
|---|---|
| P₂O₅ | 0 bis 5, 0 |
| V₂O₅ | 0 bis 6, 0 |
| Ta₂O₅ | 0 bis 5, 0 |
| Nb₂O₅ | 0 bis 5, 0. |

Das Glas und die Glaskeramik können zudem 0 bis 6,0 Gew.-% Oxid sechswertiger Elemente Me^{VI}O₃ enthalten, wobei dieses Me^{VI}O₃ insbesondere ausgewählt ist aus WO₃ und/oder MoO₃. Besonders bevorzugt enthalten das Glas und die Glaskeramik mindestens eines und insbesondere alle der folgenden Oxide Me^{VI}O₃ in den angegebenen Mengen:

| Komponente | Gew.-% |
|---|---|
| WO₃ | 0 bis 6, 0 |
| MoO₃ | 0 bis 5, 0. |

Das Glas und die Glaskeramik können ferner Oxide von weiteren f-Elementen, wie z.B. Oxide von Pr, Nd, Gd, Dy, Er und Yb und insbesondere Oxide von Er enthalten.

Weiterhin können das Glas und die Glaskeramik 0 bis 5,0 und insbesondere 0 bis 2,0 Gew.-% Fluor enthalten.

Besonders bevorzugt sind ein Glas und eine Glaskeramik, die mindestens eine und bevorzugt alle der folgenden Komponenten in den angegebenen Mengen enthalten:

| Komponente | Gew.-% |
|---|---|
| SiO₂ | 59,0 bis 77, 0 |
| Al₂O₃ | 0,3 bis 39, 0 |
| Cer, berechnet als CeO₂ | 0,7 bis 7,5 |
| Zinn, berechnet als SnO | 0,2 bis 3, 0 |
| Terbium, berechnet als Tb₄O₇ | 0 bis 2,0 |
| Li₂O | 0 bis 18, 0 |
| Me^{I}₂O | 0 bis 13,0 |
| Me^{II}O | 0 bis 22, 0 |
| Me^{III}₂O₃ | 0 bis 10,0 |
| Me^{IV}O₂ | 0 bis 4, 0 |
| Me^{V}₂O₅ | 0 bis 8,0 |
| Me^{VI}O₃ | 0 bis 6,0 |
| Fluor | 0 bis 5,0, |

wobei Me^{I}₂O, Me^{II}O, Me^{III}₂O₃, Me^{IV}O₂, Me^{V}₂O₅ und Me^{VI}O₃ insbesondere die oben angegebenen Bedeutungen haben.

In einer weiteren besonders bevorzugten Ausführungsform enthalten das Glas und die Glaskeramik mindestens eine und bevorzugt alle der folgenden Komponenten:

| | |
|---|---|
| Komponente | Gew.-% |
| SiO₂ | 59,0 bis 77, 0 |
| Al₂O₃ | 0,3 bis 39, 0 |
| Cer, berechnet als CeO₂ | 0,7 bis 7,5 |
| Zinn, berechnet als SnO | 0,2 bis 3, 0 |
| Terbium, berechnet als Tb₄O₇ | 0 bis 2, 0 |
| Li₂O | 0 bis 18,0 |
| Na₂O | 0 bis 8, 0 |
| K₂O | 0 bis 5,0 |
| Rb₂O | 0 bis 7,0 |
| Cs₂O | 0 bis 13,0 |
| MgO | 0 bis 13,0 |
| CaO | 0 bis 4,0 |
| SrO | 0 bis 3,0 |
| ZnO | 0 bis 4,0 |
| BaO | 0 bis 10,0 |
| B₂O₃ | 0 bis 5,0 |
| Y₂O₃ | 0 bis 3,0 |
| La₂O₃ | 0 bis 2,0 |
| Ga₂O₃ | 0 bis 2,0 |
| In₂O₃ | 0 bis 1,0 |
| ZrO₂ | 0 bis 4,0 |
| GeO₂ | 0 bis 4,0 |
| TiO₂ | 0 bis 5,0 |
| P₂O₅ | 0 bis 5,0 |
| V₂O₅ | 0 bis 6, 0 |
| Ta₂O₅ | 0 bis 5,0 |
| Nb₂O₅ | 0 bis 5,0 |
| WO₃ | 0 bis 6,0 |
| MoO₃ | 0 bis 5,0 |
| Er₂O₃ | 0 bis 1, 0 |
| Fluor | 0 bis 5,0. |

Die Erfindung betrifft ebenfalls Vorstufen mit entsprechender Zusammensetzung, aus denen die erfindungsgemäße Glaskeramik durch Wärmebehandlung hergestellt werden kann. Diese Vorstufen sind ein entsprechend zusammengesetztes Glas (auch als Ausgangsglas bezeichnet) und ein entsprechend zusammengesetztes Glas mit Keimen. Die Bezeichnung "entsprechender Zusammensetzung" bedeutet, dass diese Vorstufen die gleichen Komponenten in den gleichen Mengen wie die Glaskeramik enthalten, wobei die Komponenten mit Ausnahme von Fluor als Oxide berechnet werden, so wie es bei Gläsern und Glaskeramiken üblich ist.

Die Erfindung betrifft ebenfalls ein erfindungsgemäßes Glas, das Keime für die Kristallisation enthält. Durch Wärmebehandlung des erfindungsgemäßen Glases kann zunächst das erfindungsgemäße Glas mit Keimen erzeugt werden, welches seinerseits durch weitere Wärmebehandlung in die erfindungsgemäße Glaskeramik umgewandelt werden kann.

Die Erfindung betrifft auch ein Verfahren zur Herstellung des erfindungsgemäßen Glases oder der erfindungsgemäßen Glaskeramik, bei dem das Zinn in zweiwertiger Form und insbesondere als SnO eingesetzt wird.

Die Herstellung des erfindungsgemäßen Glases erfolgt insbesondere in der Weise, dass eine Mischung von geeigneten Ausgangsmaterialien, wie z.B. Carbonaten, Oxiden, Phosphaten und Fluoriden, bei Temperaturen von insbesondere 1500 bis 1800°C für 0,5 bis 10 h erschmolzen wird. Zur Erzielung einer besonders hohen Homogenität kann die erhaltene Glasschmelze in Wasser gegossen werden, um ein Glasgranulat zu bilden, und das erhaltene Granulat anschließend erneut aufgeschmolzen werden.

Die Schmelze kann dann in Formen, z.B. Stahl- oder Graphitformen, gegossen werden, um Rohlinge des Glases, sogenannte Massivglasrohlinge oder monolithische Rohlinge, zu erzeugen. Üblicherweise werden diese monolithischen Rohlinge zunächst entspannt, z.B. indem sie 5 bis 120 min bei 450 bis 600°C gehalten werden.

Es ist ebenfalls möglich, die Schmelze erneut in Wasser zu geben, um ein Granulat herzustellen. Dieses Granulat kann dann nach Mahlen und gegebenenfalls Zugabe weiterer Komponenten zu einem Rohling, einem sogenannten Pulverpressling, gepresst werden. Schließlich kann das Glas nach Granulierung auch zu einem Pulver verarbeitet werden.

Aus dem Glas kann dann durch Wärmebehandlung das Glas mit Keimen erzeugt werden. Dies wird auch als Keimbildungsprozess bezeichnet. Die Erfindung ist daher ebenfalls auf ein Verfahren zur Herstellung des Glases mit Keimen gerichtet, bei dem das Glas einer Wärmebehandlung bei einer Temperatur von 450 bis 600°C und insbesondere 500 bis 550°C für eine Dauer von insbesondere 5 bis 120 min und vorzugsweise 10 bis 60 min unterworfen wird.

Aus dem Glas mit Keimen kann dann durch Wärmebehandlung die erfindungsgemäße Glaskeramik gebildet werden. Die Erfindung ist daher ebenfalls auf ein Verfahren zur Herstellung der erfindungsgemäßen Glaskeramik gerichtet, bei dem das Glas, insbesondere das Glas mit Keimen, mindestens einer Wärmebehandlung bei einer Temperatur von 700 bis 950°C für eine Dauer von insbesondere 5 bis 120 min und vorzugsweise 10 bis 60 min unterworfen wird.

Das erfindungsgemäße Glas oder das erfindungsgemäße Glas mit Keimen können z.B. in Form eines Massivglasrohlings oder eines Pulverpresslings der mindestens einen Wärmebehandlung unterzogen werden.

Die im erfindungsgemäßen Verfahren durchgeführte mindestens eine Wärmebehandlung kann auch im Rahmen eines Heißpressens, insbesondere eines Massivglasrohlings, oder im Rahmen eines Aufsinterns, insbesondere eines Pulvers, erfolgen.

Somit betrifft die Erfindung in einer bevorzugten Ausführungsform ein Verfahren zur Herstellung der erfindungsgemäßen Glaskeramik, bei dem
(a) Pulver des erfindungsgemäßen Glases, gegebenenfalls nach Zugabe weiterer Komponenten, wie z.B. anderer Gläser, Glaskeramiken und/oder Presshilfsmitteln, zu einem Pulverpressling gepresst wird, und
(b) der Pulverpressling einer Wärmebehandlung bei einer Temperatur von 700 bis 950°C für eine Dauer von insbesondere 5 bis 120 min unterworfen wird.

In einer weiteren bevorzugten Ausführungsform betrifft die Erfindung ein Verfahren zur Herstellung der erfindungsgemäßen Glaskeramik, bei dem
(a') Schmelze des Glases zu einem Glasrohling geformt wird, insbesondere durch Gießen in eine Form, und
(b') der Glasrohling einer Wärmebehandlung bei einer Temperatur von 700 bis 900°C für eine Dauer von insbesondere 5 bis 120 min unterworfen wird.

In beiden bevorzugten Ausführungsformen des erfindungsgemäßen Verfahrens kann gegebenenfalls vor der Wärmebehandlung in Schritt (b) oder (b') eine Keimbildung vorgenommen werden.

Die Erfindung betrifft ferner ein erfindungsgemäßes Glas und eine erfindungsgemäße Glaskeramik, die eine weißlich-blaue Fluoreszenzfarbe im CIE-Farbraum aufweisen.

Die erfindungsgemäßen Gläser und Glaskeramiken mit Gehalt an Cer und Zinn eignen sich insbesondere als Mischungskomponenten zur Einstellung der Fluoreszenzeigenschaften anderer Gläser und Glaskeramiken. Ein Glas oder eine Glaskeramik, die das erfindungsgemäße Glas oder die erfindungsgemäße Glaskeramik mit Gehalt an Cer und Zinn enthalten, stellen daher einen weiteren Gegenstand der Erfindung dar. Dabei sind ein Glas und eine Glaskeramik besonders bevorzugt, die das erfindungsgemäße Glas oder die erfindungsgemäße Glaskeramik mit Gehalt an Cer und Zinn in einer Menge von 0,1 bis 50 Gew.-%, insbesondere 0,2 bis 40 Gew.-%, bevorzugt 0,5 bis 30 Gew.-%, besonders bevorzugt 1 bis 20 Gew.-% und ganz besonders bevorzugt 5 bis 10 Gew.-% enthalten.

Das erfindungsgemäße Glas oder die erfindungsgemäße Glaskeramik mit Gehalt an Cer und Zinn können insbesondere als Komponente eines anorganisch-anorganischen Komposits oder in Kombination mit einer Vielzahl von anderen Gläsern und/oder Glaskeramiken verwendet werden, wobei die Komposite oder Kombinationen insbesondere als Dentalmaterialien eingesetzt werden können. Besonders bevorzugt können die Komposite oder Kombinationen in Form von Sinterrohlingen vorliegen. Beispiele anderer Gläser und Glaskeramiken zur Herstellung von anorganisch-anorganischen Kompositen und von Kombinationen sind in DE 43 14 817 A1, DE 44 23 793 C1, DE 44 23 794 C1, DE 44 28 839 A1, DE 196 47 739 A1, DE 197 25 552 A1, DE 100 31 431 A1, EP 0 827 941 A1, EP 0 916 625 A1, WO 00/34196 A2, EP 1 505 041 A1, EP 1 688 398 A1, EP 2 287 122 A1, EP 2 377 831 A1, EP 2 407 439 A1, WO 2013/053863 A2, WO 2013/053864 A2, WO 2013/053865 A2, WO 2013/053866 A2, WO 2013/053867 A2, WO 2013/053868 A2, WO 2013/164256 A1, WO 2014/170168 A1, WO 2014/170170 A2, WO 2015/067643 A1, WO 2015/155038 A1, WO 2015/173394 A1, WO 2016/120146 A1, WO 2017/032745 A1, WO 2017/055010 A1 offenbart. Diese Gläser und Glaskeramiken gehören zur Silikat-, Borat-, Phosphat- oder Alumosilikat-Gruppe. Bevorzugte Gläser und Glaskeramiken sind vom SiO₂-Al₂O₃-K₂O-Typ (mit kubischen oder tetragonalen Leucit-Kristallen), SiO₂-B₂O₃-Na₂O-Typ, Alkali-Silikat-Typ, Alkali-Zink-Silikat-Typ, Silico-Phosphat-Typ und/oder SiO₂-ZrO₂-Typ. Besonders bevorzugt sind Lithiumsilikat-Glaskeramiken und insbesondere Glaskeramiken, die Lithiummetasilikat oder Lithiumdisilikat als Hauptkristallphase und gegebenenfalls weitere Kristallphasen wie Apatit, Diopsid, Quarz und/oder Wollastonit enthalten, sowie Glaskeramiken, die SiO₂, insbesondere in Form von Tiefquarz, als Hauptkristallphase enthalten. Durch Vermischen derartiger Gläser oder Glaskeramiken mit den erfindungsgemäßen Gläsern und/oder Glaskeramiken mit Gehalt an Cer und Zinn können insbesondere die Fluoreszenzeigenschaften in gewünschter Weise eingestellt werden.

Die erfindungsgemäßen Glaskeramiken und die erfindungsgemäßen Gläser, insbesondere in Form von Kompositen und Kombinationen, liegen insbesondere in Form von Pulvern, Granulaten oder Rohlingen in beliebiger Form und Größe, z.B. monolithischen Rohlingen, wie Plättchen, Quadern oder Zylindern, oder Pulverpresslingen, in ungesinterter, teilgesinterter oder dichtgesinterter Form, vor. In diesen Formen können sie einfach weiterverarbeitet werden, z.B. zu dentalen Restaurationen. Sie können aber auch in Form von dentalen Restaurationen, wie Inlays, Onlays, Kronen, Teilkronen, Brücken, Veneers, Schalen oder Abutments, vorliegen.

Aus den erfindungsgemäßen Glaskeramiken und den erfindungsgemäßen Gläsern, insbesondere in Form von Kompositen und Kombinationen, können dentale Restaurationen, wie Inlays, Onlays, Kronen, Teilkronen, Brücken, Veneers, Schalen oder Abutments, hergestellt werden. Die Erfindung betrifft daher deren Verwendung als Dentalmaterial und insbesondere deren Verwendung zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1150°C und insbesondere 700 bis 1000°C erfolgt. Weiter ist es bevorzugt, das Verpressen bei einem Druck von 10 bis 30 bar durchzuführen. Beim Verpressen wird durch viskoses Fließen des eingesetzten Materials die gewünschte Formänderung erreicht. Es können für das Verpressen das erfindungsgemäße Glas und das erfindungsgemäße Glas mit Keimen sowie bevorzugt die erfindungsgemäße Glaskeramik verwendet werden. Dabei können die erfindungsgemäßen Gläser und Glaskeramiken insbesondere in Form von Rohlingen in beliebiger Form und Größe, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das erfindungsgemäße Glas, das erfindungsgemäße Glas mit Keimen sowie die erfindungsgemäße Glaskeramik verwendet werden. Dabei können die erfindungsgemäßen Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt die erfindungsgemäße Glaskeramik verwendet. Die erfindungsgemäße Glaskeramik kann auch in einer noch nicht vollständig kristallisierten Form eingesetzt werden, die durch Wärmebehandlung bei niedrigerer Temperatur erzeugt wurde. Dies bietet den Vorteil, dass eine leichtere maschinelle Bearbeitung und damit der Einsatz von einfacheren Apparaten zur maschinellen Bearbeitung möglich sind. Nach der maschinellen Bearbeitung eines solchen teilkristallisierten Materials wird dieses regelmäßig einer weiteren Wärmebehandlung unterzogen, um eine weitere Kristallisation hervorzurufen.

Die erfindungsgemäßen Glaskeramiken und die erfindungsgemäßen Gläser, insbesondere in Form von Kompositen und Kombinationen, eignen sich allerdings auch als Beschichtungsmaterial von z.B. Keramiken, Glaskeramiken und Metallen. Die Erfindung ist daher ebenfalls auf die Verwendung der erfindungsgemäßen Gläser oder der erfindungsgemäßen Glaskeramiken zur Beschichtung von insbesondere Keramiken, Glaskeramiken und Metallen gerichtet.

Die Erfindung betrifft auch ein Verfahren zur Beschichtung von Keramiken, Glaskeramiken und Metallen, bei dem erfindungsgemäße Glaskeramik oder erfindungsgemäßes Glas, insbesondere in Form von Kompositen und Kombinationen, auf die Keramik, die Glaskeramik oder das Metall aufgebracht und einer Temperatur von mindestens 600°C ausgesetzt wird.

Dies kann insbesondere durch Aufsintern und bevorzugt durch Aufpressen erfolgen. Beim Aufsintern wird die Glaskeramik oder das Glas in üblicher Weise, z.B. als Pulver, auf das zu beschichtende Material, wie Keramik, Glaskeramik oder Metall, aufgebracht und anschließend gesintert. Bei dem bevorzugten Aufpressen wird erfindungsgemäße Glaskeramik oder erfindungsgemäßes Glas, z.B. in Form von Pulverpresslingen oder monolithischen Rohlingen, bei einer erhöhten Temperatur, von z.B. 700 bis 1150°C und insbesondere 700 bis 1000°C, und unter Anwendung von Druck, z.B. 10 bis 30 bar, aufgepresst. Hierzu können insbesondere die in der EP 231 773 beschriebenen Verfahren und der dort offenbarte Pressofen eingesetzt werden. Geeignete kommerzielle Öfen sind die Öfen vom Typ Programat von Ivoclar Vivadent AG, Liechtenstein.

Aufgrund der vorstehend geschilderten Eigenschaften der erfindungsgemäßen Glaskeramiken und der erfindungsgemäßen Gläser eignen sich diese insbesondere zum Einsatz in der Zahnheilkunde. Gegenstand der Erfindung ist daher auch die Verwendung der erfindungsgemäßen Glaskeramiken oder der erfindungsgemäßen Gläser, insbesondere in Form von Kompositen und Kombinationen, als Dentalmaterial und insbesondere zur Herstellung dentaler Restaurationen oder als Beschichtungsmaterial für dentale Restaurationen, wie Kronen, Brücken und Abutments.

Die Erfindung betrifft mithin auch ein Verfahren zur Herstellung einer dentalen Restauration, insbesondere Inlay, Onlay, Krone, Teilkrone, Brücke, Veneer, Schale oder Abutment, bei dem der erfindungsgemäßen Glaskeramik oder dem erfindungsgemäßen Glas, insbesondere in Form von Kompositen und Kombinationen, durch Verpressen, Sintern oder maschinelle Bearbeitung, insbesondere im Rahmen eines CAD/CAM-Verfahrens, die Form der gewünschten dentalen Restauration gegeben wird.

Die Erfindung wird im Folgenden anhand von sie nicht beschränkenden Beispielen näher erläutert.

### Beispiele

Es wurden insgesamt 17 erfindungsgemäße Gläser mit den in der Tabelle I angegebenen Zusammensetzungen hergestellt, wobei sich die Oxidationsstufen der angegebenen Oxide auf die Oxidationsstufen der eingesetzten Rohstoffe beziehen. Die Gläser wurden gemäß Tabelle II zu Glaskeramiken kristallisiert. Dabei bedeuten T_{g} Glasübergangstemperatur, bestimmt mittels DSC T_{S} und t_{S} angewendete Temperatur und Zeit zum Erschmelzen T_{N} und t_{N} angewendete Temperatur und Zeit für Keimbildung T_{C} und t_{C} angewendete Temperatur und Zeit für Kristallisation T_{Sinter} und t_{Sinter} angewendete Temperatur und Zeit für Sintern.

In den Beispielen wurden zunächst Ausgangsgläser mit den in der Tabelle I angegebenen Zusammensetzungen im 100 bis 200 g Maßstab aus üblichen Rohstoffen bei der Temperatur T_{S} für eine Dauer t_{S} erschmolzen. Durch Eingießen der erschmolzenen Ausgangsgläser in Wasser wurden Glasfritten hergestellt. Für die Weiterverarbeitung der Glasfritten wurden die nachstehend angegebenen vier Verfahrensvarianten A) bis D) benutzt:

### A) Herstellung gesinterter Pulverpresslinge

In den Beispielen 1 bis 3 (erfindungsgemäß) und 4 (Vergleich) wurde visuell die Fluoreszenz der erhaltenen Glasfritten unter einer UV-Lampe bestimmt. Dabei zeigten alle Glasfritten eine Fluoreszenz.

Danach wurden die erhaltenen Glasfritten in einer Zirkonoxidmühle auf eine Korngröße von < 112 µm aufgemahlen. Ca. 4 g dieser Pulver wurden zu zylinderförmigen Rohlingen verpresst und einer Wärmebehandlung bei der Temperatur T_{N} für eine Dauer t_{N} unterworfen, wodurch Keimbildung stattfinden konnte. Anschließend wurden die Rohlinge im Vakuum in einem Sinterofen (Programat^{®} von Ivoclar Vivadent AG) bei einer Temperatur T_{Sinter} und einer Haltezeit von t_{Sinter} zu dichten glaskeramischen Körpern gesintert. Die Fluoreszenz der so erhaltenen glaskeramischen Körper wurde visuell unter einer UV-Lampe bestimmt. Dabei zeigten alle Körper eine Fluoreszenz.

Die Beispiele wurden wiederholt, wobei das Sintern in Luft erfolgte. Während bei den erfindungsgemäßen Beispielen 1 bis 3 eine gegenüber dem Sintern im Vakuum lediglich etwas geminderte Fluoreszenz erhalten wurde, konnte beim Vergleichsbeispiel 4 (kein Zinn) keine nennenswerte Fluoreszenz mehr beobachtet werden.

### B) Massivglasblöcke

In den Beispielen 5 bis 15 (erfindungsgemäß) und 16 (Vergleich) wurden die erhaltenen Glasfritten erneut bei der Temperatur T_{S} für eine Dauer t_{S} aufgeschmolzen. Die erhaltenen Schmelzen des Ausgangsglases wurden anschließend in eine Graphitform gegossen, um Massivglasblöcke zu erzeugen. Die Glasmonolithe wurden daraufhin bei der Temperatur T_{N} für eine Dauer t_{N} entspannt, wodurch Keimbildung stattfinden konnte. Anschließend wurde visuell die Fluoreszenz der Glasmonolithe unter einer UV-Lampe bestimmt. Dabei zeigten alle Glasmonolithe mit Ausnahme des Vergleichsbeispiels 16 (kein Zinn) eine Fluoreszenz.

In den Beispielen 6 bis 15 (erfindungsgemäß) und 16 (Vergleich) wurden die Glasmonolithe dann für eine Dauer t_{C} auf eine Temperatur T_{C} erwärmt, um Glaskeramiken zu bilden. Die Fluoreszenz der erhaltenen Glaskeramiken wurde wiederum visuell unter einer UV-Lampe bestimmt. Alle Glaskeramiken mit Ausnahme des Vergleichsbeispiels 16 (kein Zinn) zeigten eine Fluoreszenz.

### C) Glasfritten

In den Beispielen 17 und 18 wurde visuell die Fluoreszenz der erhaltenen Glasfritten unter einer UV-Lampe bestimmt. Dabei zeigten alle Glasfritten eine Fluoreszenz.

### D) Erfindungsgemäßes Glas als Mischungskomponente

Eine gemäß Beispiel 19 erhaltene Glasfritte wurde aufgemahlen und einem aufgemahlenen Glaspulver mit einer Zusammensetzung gemäß der WO 2015/173394 A1 in unterschiedlichen Mengen (5, 10, 20 und 30 Gew.-% bezogen auf die Mischung) zugemischt. Jeweils ca. 4 g dieser Mischungen wurden anschließend zu zylinderförmigen Rohlingen verpresst und in einem Sinterofen (Programat^{®} von Ivoclar Vivadent AG) bei einer Temperatur von 860°C und einer Haltezeit von 60 min zu dichten glaskeramischen Körpern gesintert. Anschließend wurde visuell die Fluoreszenz unter einer UV-Lampe bestimmt. Dabei zeigten alle erhaltenen Körper eine Fluoreszenz.

**Tabelle I**

| **Beispiel** | **1** | **2** | **3** | **4**** | **5** | **6** | **7** | **8** | **9** | **10** |
|---|---|---|---|---|---|---|---|---|---|---|
| Zusammen setzung | Gew.-% | Gew. -% | Gew.-% | Gew. -% | Gew. -% | Gew. -% | Gew.-% | Gew. -% | Gew. -% | Gew. -% |
| SiO₂ | 73, 8 | 73,4 | 70, 8 | 73,3 | 63,7 | 73,2 | 73,2 | 73,4 | 73, 8 | 76,2 |
| Al₂O₃ | 2, 6 | 2, 6 | 2, 5 | 2, 6 | 5, 4 | 0, 5 | 2, 6 | 2, 6 | 2, 6 | 2, 7 |
| CeO₂ | 1, 3 | 1, 8 | 0, 9 | - | 7, 3 | 4, 4 | 1, 8 | 1, 8 | 1, 8 | 0, 9 |
| Ce₂O₃* | - | - | - | 2, 4 | - | - | - | - | - | - |
| SnO | 0, 5 | 0, 5 | 0, 3 | - | 2, 8 | 1, 4 | 0, 5 | 0, 5 | 0, 5 | 0, 2 |
| Li₂O | 9, 0 | 8, 9 | 7, 9 | 8, 9 | 15, 8 | 7, 9 | 8, 9 | 8, 9 | 8, 6 | 8, 9 |
| Na₂O | - | | | - | - | | | | | |
| K₂O | 3, 2 | 3, 2 | 3, 4 | 3, 2 | 5, 0 | 3, 5 | 3, 2 | 3, 2 | 3, 2 | 3, 3 |
| MgO | 2, 4 | 2, 4 | 1, 7 | 2, 4 | - | 1, 7 | 2, 4 | 2, 4 | 2, 7 | 2, 8 |
| CaO | - | - | 3, 0 | - | - | 3, 0 | - | - | - | - |
| SrO | - | - | - | - | - | - | - | - | - | - |
| ZnO | 3, 5 | 3, 5 | - | 3, 5 | - | - | 3, 5 | 3, 5 | 0, 7 | 0, 7 |
| B₂O₃ | - | - | - | - | - | - | - | - | 0, 6 | 0, 6 |
| Y₂O₃ | - | - | - | - | - | - | - | - | - | - |
| La₂O₃ | - | - | - | - | - | - | - | - | - | - |
| GeO₂ | - | - | 1, 4 | - | - | - | - | - | - | - |
| ZrO₂ | - | - | 1, 0 | - | - | - | - | - | - | - |
| P₂O₅ | 3, 3 | 3, 3 | 3, 4 | 3, 3 | - | 3, 6 | 3, 3 | 3, 3 | 3, 2 | 3, 3 |
| V₂O₅ | - | - | - | - | - | - | 0, 1 | - | - | - |
| Ta₂O₅ | - | - | 3, 7 | - | - | - | - | - | 1, 9 | - |
| Er₂O₃ | - | - | - | - | - | - | 0, 1 | - | - | - |
| Tb₄O₇ | 0, 4 | 0, 4 | - | 0, 4 | - | 0, 8 | 0, 4 | 0, 4 | 0, 4 | 0, 4 |
| F | - | - | - | - | - | - | - | - | - | - |
| Σ | 100,0 | 100,0 | 100, 0 | 100,0 | 100,0 | 100,0 | 100,0 | 100,0 | 100,0 | 100,0 |

**Tabelle I**

| **Beispiel** | **11** | **12** | **13** | **14** | **15** | **16**** | **17** | **18** | **19** |
|---|---|---|---|---|---|---|---|---|---|
| Zusammen setzung | Gew.-% | Gew.-% | Gew.-% | Gew.-% | Gew.-% | Gew.-% | Gew.-% | Gew.-% | Gew.-% |
| SiO₂ | 74, 1 | 73, 8 | 73, 8 | 71, 8 | 70, 8 | 76, 5 | 64, 6 | 63, 9 | 73, 9 |
| Al₂O₃ | 2, 6 | 2, 6 | 2, 6 | 2, 6 | 2, 5 | 2, 7 | 4, 0 | 5, 0 | 0, 5 |
| CeO₂ | 1, 8 | 1, 8 | 1, 8 | 4, 4 | 0, 9 | 0, 9 | 1, 9 | 1,7 | 4, 4 |
| Ce₂O₃* | - | - | - | - | - | - | - | - | - |
| SnO | 0, 5 | 0, 5 | 0, 5 | 0, 5 | 0, 3 | - | 0, 7 | 0, 7 | 1, 4 |
| Li₂O | 8, 7 | 8, 6 | 8, 9 | 8, 7 | 7, 9 | 9, 2 | 16, 0 | 3, 2 | 8, 0 |
| Na₂O | - | - | - | - | - | - | - | 7, 4 | - |
| K₂O | 3, 2 | 3, 2 | 3, 2 | 3, 1 | 3, 4 | 3, 3 | 3, 8 | 4, 6 | 3, 5 |
| MgO | 2, 7 | 2, 7 | 2, 4 | 2, 3 | 1, 7 | 3, 0 | 1, 8 | 0, 5 | 1, 7 |
| CaO | - | - | - | - | 3, 0 | - | 3, 3 | 1, 8 | 3, 0 |
| SrO | - | - | - | - | - | - | - | 2, 4 | - |
| ZnO | 0, 7 | 0, 7 | 35 | 3, 4 | - | 0, 7 | - | 3, 5 | - |
| B₂O₃ | 0, 6 | 0, 6 | - | - | - | - | - | 4, 0 | - |
| Y₂O₃ | - | 1 9 | - | - | - | - | - | - | - |
| La₂O₃ | 1, 4 | - | - | - | - | - | - | - | - |
| GeO₂ | - | - | - | - | 1, 4 | - | - | - | - |
| ZrO₂ | - | - | - | - | 1, 0 | - | - | - | - |
| P₂O₅ | 3, 3 | 3, 2 | 3, 3 | 3, 2 | 3, 4 | 3, 3 | 3, 9 | - | 3, 6 |
| V₂O₅ | - | - | - | - | - | - | - | - | - |
| Ta₂O₅ | - | - | - | - | 3, 7 | - | - | - | - |
| Er₂O₃ | - | - | - | - | - | - | - | - | - |
| Tb₄O₇ | 0, 4 | 0, 4 | - | - | - | 0, 4 | - | - | - |
| F | - | - | - | - | - | - | - | 1, 3 | - |
| Σ | 100,0 | 100, 0 | 100,0 | 100,0 | 100,0 | 100,0 | 100,0 | 100,0 | 100,0 |

| | | | | | | | | | |
|---|---|---|---|---|---|---|---|---|---|
| * eingesetzt als Cer(III)-acetylacetonat; ** Vergleich | | | | | | | | | |

**Tabelle II**

| **Beispiel** | **1** | **2** | **3** | **4**** | **5** | **6** | **7** | **8** | **9** | **10** |
|---|---|---|---|---|---|---|---|---|---|---|
| T_{g} [°C] | | | | | 469 | 488 | | | | |
| T_{S} [°C] | 1600 | 1600 | 1600 | 1600 | 1650 | 1650 | 1600 | 1600 | 1600 | 1600 |
| t_{S} [min] | 120 | 120+120 | 60 | 120 | 240 | 60 | 120+120 | 120+120 | 120+120 | 120 |
| T_{N} [°C] | 500 | 500 | 500 | 500 | 490 | 520 | 500 | 500 | 500 | 500 |
| t_{N} [min] | 60 | 20 | 20 | 60 | 120 | 10 | 60 | 60 | 60 | 60 |
| T_{C} [°C] | - | - | - | - | - | 850 | 820 | 820 | 820 | 820 |
| t_{C} [min] | - | - | - | - | - | 10 | 30 | 30 | 30 | 30 |
| T_{Sinter} [°C] | 860 | 860 | 860 | 860 | - | - | - | - | - | - |
| t_{Sinter} [min] | 30 | 60 | 60 | 30 | - | - | - | - | - | - |
| Fluoreszenz Glas | blau | stark blau | blauweiß | blau | blau | blauweiß | blau | stark blau | blau | blau |
| Fluoreszenz Glaskeramik | blau | blau | blauweiß | blau | | blauweiß | blau | stark blau | blau | blau |

**Tabelle II**

| **Beispiel** | **11** | **12** | **13** | **14** | **15** | **16**** | **17** | **18** |
|---|---|---|---|---|---|---|---|---|
| T_{g} [°C] | | | | | | | 435 | 445 |
| T_{S} [°C] | 1600 | 1600 | 1600 | 1600 | 1600 | 1600 | 1600 | 1600 |
| t_{S} [min] | 120 | 120 | 120+120 | 120+120 | 60 | 120 | 120 | 60 |
| T_{N} [°C] | 500 | 500 | 490 | 500 | 500 | 500 | - | - |
| t_{N} [min] | 60 | 60 | 10 | 10 | 10 | 60 | - | - |
| T_{C} [°C] | 820 | 820 | 820 | 820 | 810 | 820 | - | - |
| t_{C} [min] | 30 | 30 | 30 | 30 | 60 | 30 | - | - |
| T_{Sinter} [°C] | - | - | - | - | - | - | - | - |
| t_{Sinter} [min] | - | - | - | - | - | - | - | - |
| Fluoreszenz Glas | blau | blau | stark blau | stark blau | blauweiß | keine | blau | blau |
| Fluoreszenz Glaskeramik | blau | blau | stark blau | schwach blau | blauweiß | keine | | |

| | | | | | | | | |
|---|---|---|---|---|---|---|---|---|
| ** Vergleich | | | | | | | | |

## Patentansprüche

1. Glas oder Glaskeramik mit Gehalt an Cer und Zinn, die die folgenden Komponenten enthalten:
| Komponente | Gew.-% |
|---|---|
| SiO₂ | 59,0 bis 80, 0 |
| Al₂O₃ | 0,1 bis 42, 0 |
| Cer, berechnet als CeO₂ | 0,5 bis 10,0 |
| Zinn, berechnet als SnO | 0,1 bis 4, 0 |
| B₂O₃ | 0 bis 5,0 |
| weiteres Oxid vierwertiger | |
| Elemente Me^{IV}O₂ | 0 bis 4, 0, |
wobei der Begriff "weiteres Oxid vierwertiger Elemente Me^{IV}O₂" vierwertige Oxide mit Ausnahme von SiO₂, CeO₂, SnO₂ und TiO₂ bezeichnet und dieses Me^{IV}O₂ insbesondere ausgewählt ist aus ZrO₂ und/oder GeO₂ und
wobei das molare Verhältnis von Cer, berechnet als CeO₂, zu Zinn, berechnet als SnO, im Bereich von 10:1 bis 1:1 liegt.

2. Glas oder Glaskeramik nach Anspruch 1, die 59,0 bis 77,0, insbesondere 59,0 bis 76,0, bevorzugt 64,0 bis 75,0 und besonders bevorzugt 70,0 bis 74,0 Gew.-% SiO₂ enthalten.

3. Glas oder Glaskeramik nach Anspruch 1 oder 2, die 0,3 bis 39,0, insbesondere 0,5 bis 30,0, bevorzugt 1,0 bis 20,0, besonders bevorzugt 1,5 bis 10,0 und am meisten bevorzugt 2,0 bis 6,0 Gew.-% Al₂O₃ enthalten.

4. Glas oder Glaskeramik nach einem der Ansprüche 1 bis 3, die 0,7 bis 7,5, insbesondere 1,0 bis 7,0, bevorzugt 1,5 bis 5,0 und besonders bevorzugt 2,0 bis 4,0 Gew.-% Cer, berechnet als CeO₂, enthalten.

5. Glas oder Glaskeramik nach einem der Ansprüche 1 bis 4, die 0,2 bis 3,0, insbesondere 0,3 bis 2,0 und bevorzugt 0,4 bis 1,0 Gew.-% Zinn, berechnet als SnO, enthalten.

6. Glas oder Glaskeramik nach einem der Ansprüche 1 bis 5, bei denen das molare Verhältnis von Cer, berechnet als CeO₂, zu Zinn, berechnet als SnO, im Bereich von 5:1 bis 1:1, bevorzugt 3:1 bis 1:1 und besonders bevorzugt 2:1 bis 1:1 liegt.

7. Glas oder Glaskeramik nach einem der Ansprüche 1 bis 6, die 0 bis 2,0, insbesondere 0,05 bis 1,5, bevorzugt 0,1 bis 1,0 und besonders bevorzugt 0,3 bis 0,7 Gew.-% Terbium, berechnet als Tb₄O₇, enthalten.

8. Glas oder Glaskeramik nach einem der Ansprüche 1 bis 7, die 0 bis 18,0, insbesondere 1,0 bis 17,0, bevorzugt 3,0 bis 16,0 und besonders bevorzugt 7,5 bis 10,0 Gew.-% Li₂O enthalten.

9. Glas oder Glaskeramik nach einem der Ansprüche 1 bis 8, die 0 bis 10,0, insbesondere 0 bis 5,0 und bevorzugt 0 bis 1,0 Gew.-% BaO enthalten und am meisten bevorzugt im Wesentlichen frei von BaO sind.

10. Glas oder Glaskeramik nach einem der Ansprüche 1 bis 9, die mindestens eine und bevorzugt alle der folgenden Komponenten in den angegebenen Mengen enthalten:
| Komponente | Gew.-% |
|---|---|
| SiO₂ | 59,0 bis 77, 0 |
| Al₂O₃ | 0,3 bis 39, 0 |
| Cer, berechnet als CeO₂ | 0,7 bis 7,5 |
| Zinn, berechnet als SnO | 0,2 bis 3,0 |
| Terbium, berechnet als Tb₄O₇ | 0 bis 2,0 |
| Li₂O | 0 bis 18, 0 |
| Me^{I}₂O | 0 bis 13,0 |
| Me^{II}O | 0 bis 22, 0 |
| Me^{III}₂O₃ | 0 bis 10,0 |
| Me^{IV}O₂ | 0 bis 4,0 |
| Me^{V}₂O₅ | 0 bis 8,0 |
| Me^{VI}O₃ | 0 bis 6,0 |
| Fluor | 0 bis 5,0, |
wobei
Me^{I}₂O insbesondere ausgewählt ist aus Na₂O, K₂O, Rb₂O und/oder Cs₂O,
Me^{II}O insbesondere ausgewählt ist aus MgO, CaO, SrO und/oder ZnO,
Me^{III}₂O₃ insbesondere ausgewählt ist aus B₂O₃, Y₂O₃, La₂O₃, Ga₂O₃ und/oder In₂O₃,
Me^{IV}O₂ insbesondere ausgewählt ist aus ZrO₂ und/oder GeO₂, Me^{V}₂O₅ insbesondere ausgewählt ist aus P₂O₅, V₂O₅, Ta₂O₅ und/oder Nb₂O₅ und
Me^{VI}O₃ insbesondere ausgewählt ist aus WO₃ und/oder MoO₃.

11. Glas nach einem der Ansprüche 1 bis 10, das Keime für die Kristallisation enthält.

12. Glas oder Glaskeramik, die das Glas oder die Glaskeramik mit Gehalt an Cer und Zinn nach einem der Ansprüche 1 bis 11 enthalten, vorzugsweise in einer Menge von 0,1 bis 50 Gew.-%, insbesondere 0,2 bis 40 Gew.-%, bevorzugt 0,5 bis 30 Gew.-%, besonders bevorzugt 1 bis 20 Gew.-% und ganz besonders bevorzugt 5 bis 10 Gew.-%.

13. Glas oder Glaskeramik nach einem der Ansprüche 1 bis 12, wobei das Glas und die Glaskeramik in Form von einem Pulver, einem Granulat, einem Rohling oder einer dentalen Restauration vorliegen.

14. Verfahren zur Herstellung des Glases oder der Glaskeramik nach einem der Ansprüche 1 bis 13, bei dem das Zinn mindestens teilweise in zweiwertiger Form und insbesondere als SnO eingesetzt wird.

15. Verfahren zur Herstellung der Glaskeramik nach einem der Ansprüche 1 bis 10, 12 und 13, bei dem das Glas gemäß einem der Ansprüche 1 bis 13 mindestens einer Wärmebehandlung bei einer Temperatur von 700 bis 950°C für eine Dauer von insbesondere 5 bis 120 min, vorzugsweise 10 bis 60 min, unterworfen wird.

16. Verfahren nach Anspruch 15, bei dem
(a) Pulver des Glases, gegebenenfalls nach Zugabe weiterer Komponenten, zu einem Pulverpressling gepresst wird, und
(b) der Pulverpressling einer Wärmebehandlung bei einer Temperatur von 700 bis 950°C für eine Dauer von insbesondere 5 bis 120 min unterworfen wird,
oder
(a') Schmelze des Glases zu einem Glasrohling geformt wird, insbesondere durch Gießen in eine Form, und
(b') der Glasrohling einer Wärmebehandlung bei einer Temperatur von 700 bis 900°C für eine Dauer von insbesondere 5 bis 120 min unterworfen wird.

17. Verwendung des Glases oder der Glaskeramik gemäß einem der Ansprüche 1 bis 11 als Mischungskomponente zur Einstellung der Fluoreszenz eines Glases oder einer Glaskeramik.

18. Verwendung des Glases oder der Glaskeramik gemäß einem der Ansprüche 1 bis 13 als Dentalmaterial und insbesondere zur Herstellung dentaler Restaurationen.

19. Verwendung nach Anspruch 18, wobei dem Glas oder der Glas keramik durch Verpressen, Sintern oder maschinelle Bearbeitung, insbesondere im Rahmen eines CAD/CAM-Verfahrens, die Form der gewünschten dentalen Restauration, insbesondere Inlay, Onlay, Krone, Teilkrone, Brücke, Veneer, Schale oder Abutment gegeben wird.

20. Verfahren zur Herstellung einer dentalen Restauration, insbesondere Inlay, Onlay, Krone, Teilkrone, Brücke, Veneer, Schale oder Abutment, bei dem dem Glas oder der Glaskeramik gemäß einem der Ansprüche 1 bis 13 durch Verpressen, Sintern oder maschinelle Bearbeitung, insbesondere im Rahmen eines CAD/CAM-Verfahrens, die Form der gewünschten dentalen Restauration gegeben wird.

## Claims

1. Glass or glass ceramic with cerium and tin content, which comprise the following components:
| Component | wt.-% |
|---|---|
| SiO₂ | 59.0 to 80.0 |
| Al₂O₃ | 0.1 to 42.0 |
| Cerium, calculated as CeO₂ | 0.5 to 10.0 4.0 |
| Tin, calculated as SnO | 0.1 to |
| B₂O₃ | 0 to 5.0 |
| further oxide of tetravalent | |
| elements Me^{IV}O₂ | 0 to 4.0, |
wherein the term "further oxide of tetravalent elements Me^{IV}O₂" denotes tetravalent oxides with the exception of SiO₂, CeO₂, SnO₂ and TiO₂ and this Me^{IV}O₂ is selected in particular from ZrO₂ and/or GeO₂, and
wherein the molar ratio of cerium, calculated as CeO₂, to tin, calculated as SnO, lies in the range of from 10:1 to 1:1.

2. Glass or glass ceramic according to claim 1, which comprise 59.0 to 77.0, in particular 59.0 to 76.0, preferably 64.0 to 75.0 and particularly preferably 70.0 to 74.0 wt.-% SiO₂.

3. Glass or glass ceramic according to claim 1 or 2, which comprise 0.3 to 39.0, in particular 0.5 to 30.0, preferably 1.0 to 20.0, particularly preferably 1.5 to 10.0 and most preferably 2.0 to 6.0 wt.-% Al₂O₃.

4. Glass or glass ceramic according to any one of claims 1 to 3, which comprise 0.7 to 7.5, in particular 1.0 to 7.0, preferably 1.5 to 5.0, and particularly preferably 2.0 to 4.0 wt.-% cerium, calculated as CeO₂.

5. Glass or glass ceramic according to any one of claims 1 to 4, which comprise 0.2 to 3.0, in particular 0.3 to 2.0 and preferably 0.4 to 1.0 wt.-% tin, calculated as SnO.

6. Glass or glass ceramic according to any one of claims 1 to 5, in which the molar ratio of cerium, calculated as CeO₂, to tin, calculated as SnO, lies in the range of from 5:1 to 1:1, preferably 3:1 to 1:1 and particularly preferably 2:1 to 1:1.

7. Glass or glass ceramic according to any one of claims 1 to 6, which comprise 0 to 2.0, in particular 0.05 to 1.5, preferably 0.1 to 1.0, and particularly preferably 0.3 to 0.7 wt.-% terbium, calculated as Tb₄O₇.

8. Glass or glass ceramic according to any one of claims 1 to 7, which comprise 0 to 18.0, in particular 1.0 to 17.0, preferably 3.0 to 16.0, and particularly preferably 7.5 to 10.0 wt.-% Li₂O.

9. Glass or glass ceramic according to any one of claims 1 to 8, which comprise 0 to 10.0, in particular 0 to 5.0 and preferably 0 to 1.0 wt.-% BaO, and which are most preferably substantially free from BaO.

10. Glass or glass ceramic according to any one of claims 1 to 9, which comprise at least one and preferably all of the following components in the specified amounts:
| Component | wt.-% |
|---|---|
| SiO₂ | 59.0 to 77.0 |
| Al₂O₃ | 0.3 to 39.0 |
| Cerium, calculated as CeO₂ | 0.7 to7.5 |
| Tin, calculated as SnO | 0.2 to 3.0 |
| Terbium, calculated as Tb₄O₇ | 0 to 2.0 |
| Li₂O | 0 to 18.0 |
| Me^{I}₂O | 0 to 13.0 |
| | |
|---|---|
| Me^{II}O | 0 to 22.0 |
| Me^{III}₂O₃ | 0 to 10.0 |
| Me^{IV}O₂ | 0 to 4.0 |
| Me^{V}₂O₅ | 0 to 8.0 |
| Me^{VI}O₃ | 0 to 6.0 |
| Fluorine | 0 to5.0, |
wherein
Me^{I}₂O is selected in particular from Na₂O, K₂O, Rb₂O and/or Cs₂O,
Me^{II}O is selected in particular from MgO, CaO, SrO and/or ZnO,
Me^{III}₂O₃ is selected in particular from B₂O₃, Y₂O₃, La₂O₃, Ga₂O₃ and/or In₂O₃,
Me^{IV}O₂ is selected in particular from ZrO₂ and/or GeO₂,
Me^{V}₂O₅ is selected in particular from P₂O₅, V₂O₅, Ta₂O₅ and/or Nb₂O₅ and
Me^{VI}O₃ is selected in particular from WO₃ and/or MoO₃.

11. Glass according to any one of claims 1 to 10, which comprises nuclei for the crystallization.

12. Glass or glass ceramic, which comprise the glass or the glass ceramic with cerium and tin content according to any one of claims 1 to 11, preferably in an amount of from 0.1 to 50 wt.-%, in particular 0.2 to 40 wt.-%, preferably 0.5 to 30 wt.-%, particularly preferably 1 to 20 wt.-% and more preferably 5 to 10 wt.-%.

13. Glass or glass ceramic according to any one of claims 1 to 12, wherein the glass and the glass ceramic are present in the form of a powder, a granulate, a blank or a dental restoration.

14. Process for the preparation of the glass or the glass ceramic according to any one of claims 1 to 13, in which the tin is used at least partially in divalent form and in particular as SnO.

15. Process for the preparation of the glass ceramic according to any one of claims 1 to 10, 12 and 13, in which the glass according to any one of claims 1 to 13 is subjected to at least one heat treatment at a temperature of from 700 to 950°C for a duration of in particular 5 to 120 min, preferably 10 to 60 min.

16. Process according to claim 15, in which
(a) powder of the glass, optionally after the addition of further components, is pressed to form a powder compact, and
(b) the powder compact is subjected to a heat treatment at a temperature of from 700 to 950°C, for a duration of in particular 5 to 120 min,
or
(a') melt of the glass is shaped to form a glass blank, in particular by pouring into a mould, and
(b') the glass blank is subjected to a heat treatment at a temperature of from 700 to 900°C, for a duration of in particular 5 to 120 min.

17. Use of the glass or of the glass ceramic according to any one of claims 1 to 11 as blending component for setting the fluorescence of a glass or of a glass ceramic.

18. Use of the glass or of the glass ceramic according to any one of claims 1 to 13 as dental material and in particular for the preparation of dental restorations.

19. Use according to claim 18, wherein the glass or the glass ceramic is given the shape of the desired dental restoration, in particular inlay, onlay, crown, partial crown, bridge, veneer, facet or abutment, by pressing, sintering or machining, in particular in a CAD/CAM process.

20. Process for the preparation of a dental restoration, in particular inlay, onlay, crown, partial crown, bridge, veneer, facet or abutment, in which the glass or the glass ceramic according to any one of claims 1 to 13 is given the shape of the desired dental restoration by pressing, sintering or machining, in particular in a CAD/CAM process.

## Revendications

1. Verre ou vitrocéramique à teneur en cérium et étain, qui contiennent les composants suivants :
| Composant | % en poids |
|---|---|
| SiO₂ | 59,0 à 80,0 |
| Al₂O₃ | 0,1 à 42,0 |
| cérium, calculé en tant que CeO₂ | 0,5 à 10,0 |
| étain, calculé en tant que SnO | 0,1 à 4,0 |
| B₂O₃ | 0 à 5,0 |
| autre oxyde d'éléments | |
| tétravalents Me^{iv}O₂ | 0 à 4,0 |
l'expression « autre oxyde d'éléments tétravalents Me^{iv}O₂ » désignant des oxydes tétravalents à l'exclusion de SiO₂, CeO₂, SnO₂ et TiO₂ et cet Me^{iv}O₂ étant en particulier choisi parmi ZrO₂ et/ou GeO₂ et
le rapport molaire de cérium, calculé en tant que CeO₂, à étain, calculé en tant que SnO, se situant dans la plage de 10:1 à 1:1.

2. Verre ou vitrocéramique selon la revendication 1, qui contiennent 59,00 à 77,0, en particulier 59,0 à 76,0, de préférence 64,0 à 75,0 et de façon particulièrement préférée 70,0 à 74,0 % en poids de SiO₂.

3. Verre ou vitrocéramique selon la revendication 1 ou 2, qui contiennent 0,3 à 39,0, en particulier 0,5 à 30,0, de préférence 1,0 à 20,0, de façon particulièrement préférée 1,5 à 10,0 et de façon tout particulièrement préférée 2,0 à 6,0 % en poids d'Al₂O₃.

4. Verre ou vitrocéramique selon l'une quelconque des revendications 1 à 3, qui contiennent 0,7 à 7,5, en particulier 1,0 à 7,0, de préférence 1,5 à 5,0 et de façon particulièrement préférée 2,0 à 4,0 % en poids de cérium, calculé en tant que CeO₂.

5. Verre ou vitrocéramique selon l'une quelconque des revendications 1 à 4, qui contiennent 0,2 à 3,0, en particulier 0,3 à 2,0 et de préférence 0,4 à 1,0 % en poids d'étain, calculé en tant que SnO.

6. Verre ou vitrocéramique selon l'une quelconque des revendications 1 à 5, dans lesquels le rapport molaire de cérium, calculé en tant que CeO₂, à étain, calculé en tant que SnO, se situe dans la plage de 5:1 à 1:1, de préférence 3:1 à 1:1 et de façon particulièrement préférée 2:1 à 1:1.

7. Verre ou vitrocéramique selon l'une quelconque des revendications 1 à 6, qui contiennent 0 à 2,0, en particulier 0,05 à 1,5, de préférence 0,1 à 1,0 et de façon particulièrement préférée 0,3 à 0,7 % en poids de terbium, calculé en tant que Tb₄O₇.

8. Verre ou vitrocéramique selon l'une quelconque des revendications 1 à 7, qui contiennent 0 à 18,0, en particulier 1,0 à 17,0, de préférence 3,0 à 16,0 et de façon particulièrement préférée 7,5 à 10,0 % en poids de Li₂O.

9. Verre ou vitrocéramique selon l'une quelconque des revendications 1 à 8, qui contiennent 0 à 10,0, en particulier 0 à 5,0 et de préférence 0 à 1,0 % en poids de BaO et de façon tout particulièrement préférée sont essentiellement exempts de BaO.

10. Verre ou vitrocéramique selon l'une quelconque des revendications 1 à 9, qui contiennent au moins un et de préférence la totalité des composants suivants en les quantités indiquées :
| Composant | % en poids |
|---|---|
| SiO₂ | 59,0 à 77,0 |
| Al₂O₃ | 0,3 à 39,0 |
| cérium, calculé en tant que CeO₂ | 0,7 à 7,5 |
| étain, calculé en tant que SnO | 0,2 à 3,0 |
| terbium, calculé en tant que Tb₄O₇ | 0 à 2,0 |
| Li₂O | 0 à 18,0 |
| Me^{I}₂O | 0 à 13,0 |
| Me^{II}O | 0 à 22,0 |
| Me^{III}₂O₃ | 0 à 10,0 |
| Me^{IV}O₂ | 0 à 4,0 |
| Me^{V}O₅ | 0 à 8,0 |
| Me^{VI}O₃ | 0 à 6,0 |
| fluor | 0 à 5,0, |
où
Me^{I}₂O est choisi en particulier parmi Na₂O, K₂O, Rb₂O et/ou Cs₂O,
Me^{II}O est choisi en particulier parmi MgO, CaO, SrO et/ou ZnO,
Me^{III}₂O₃ est choisi en particulier parmi B₂O₃, Y₂O₃, La₂O₃, Ga₂O₃ et/ou In₂O₃,
Me^{IV}O₂ est choisi en particulier parmi ZrO₂ et/ou GeO₂,
Me^{V}₂O₅ est choisi en particulier parmi P₂O₅, V₂O₅, Ta₂O₅ et/ou Nb₂O₅ et
Me^{VI}O₃ est choisi en particulier parmi WO₃ et/ou MoO₃.

11. Verre selon l'une quelconque des revendications 1 à 10, qui contient des germes pour la cristallisation.

12. Verre ou vitrocéramique qui contiennent le verre ou la vitrocéramique à teneur en cérium et étain selon l'une quelconque des revendications 1 à 11, de préférence en une quantité de 0,1 à 50 % en poids, en particulier 0,2 à 40 % en poids, de préférence 0,5 à 30 % en poids, de façon particulièrement préférée 1 à 20 % en poids, et de façon tout particulièrement préférée 5 à 10 % en poids.

13. Verre ou vitrocéramique selon l'une quelconque des revendications 1 à 12, le verre et la vitrocéramique se trouvant sous forme d'une poudre, d'un produit granulé, d'une ébauche ou d'une restauration dentaire.

14. Procédé pour la production du verre ou de la vitrocéramique selon l'une quelconque des revendications 1 à 13, dans lequel l'étain est utilisé au moins en partie sous forme divalente et en particulier sous forme de SnO.

15. Procédé pour la production de la vitrocéramique selon l'une quelconque des revendications 1 à 10, 12 et 13, dans lequel le verre selon l'une quelconque des revendications 1 à 13 est soumis au moins à un traitement thermique à une température de 700 à 950 °C pendant une durée d'en particulier 5 à 120 minutes, de préférence 10 à 60 minutes.

16. Procédé selon la revendication 15, dans lequel
(a) de la poudre du verre, éventuellement après addition de composants supplémentaires, est pressée en une ébauche en poudre pressée, et
(b) l'ébauche en poudre pressée est soumise à un traitement thermique à une température de 700 à 950°C pendant une durée d'en particulier 5 à 120 minutes,
ou
(a') la masse fondue du verre est mise en forme en une ébauche en verre, en particulier par coulée dans un moule, et
(b') l'ébauche en verre est soumise à un traitement thermique à une température de 700 à 900 °C pendant une durée d'en particulier 5 à 120 minutes,

17. Utilisation du verre ou de la vitrocéramique selon l'une quelconque des revendications 1 à 11 en tant que composant de mélange pour l'établissement de la fluorescence d'un verre ou d'une vitrocéramique.

18. Utilisation du verre ou de la vitrocéramique selon l'une quelconque des revendications 1 à 13 en tant que matériau dentaire et en particulier pour la fabrication de restaurations dentaires.

19. Utilisation selon la revendication 18, dans laquelle la forme de la restauration dentaire souhaitée, en particulier inlay, onlay, couronne, couronne partielle, bridge, facette, coque ou pilier, est donnée au verre ou à la vitrocéramique par pressage, frittage ou usinage mécanique en particulier dans le cadre d'un procédé CAD/CAM.

20. Procédé pour la fabrication d'une restauration dentaire, en particulier inlay, onlay, couronne, couronne partielle, bridge, facette, coque ou pilier, dans lequel la forme de la restauration dentaire souhaitée est donnée au verre ou à la vitrocéramique selon l'une quelconque des revendications 1 à 13 par pressage, frittage ou usinage mécanique, en particulier dans le cadre d'un procédé CAD/CAM.
